# EUROPEAN PATENT SPECIFICATION

(11) **EP 3 383 287 B1**
(45) Date of publication and mention of the grant of the patent: **05.02.2020**
(21) Application number: 16805456.7
(22) Date of filing: 02.12.2016
(51) Int. Cl.: A61B 17/32, A61B 17/00, A61B 90/00

(54) **SURGICAL PROBE**
CHIRURGISCHE SONDE
SONDE CHIRURGICALE

(30) Priority: 03.12.2015 EP 15197865; 02.08.2016 EP 16182478
(43) Date of publication of application: 10.10.2018
(73) Proprietor: Falke Medical Sagl, 6830 Chiasso (CH)
(72) Inventor: PAJARDI, Giorgio, 20123 Milano (IT); EPPER, Martin, 8047 Zürich (CH); CHERUBINO, Tommaso, 21100 Varese (IT)
(74) Representative: Dittmann, Rolf
(86) International application number: PCT/EP2016/079687
(87) International publication number: WO 2017/093548

(56) References cited:
- WO-A2-2010/065219
- WO-A2-2011/017665
- US-A1- 2008 109 021
- US-A1- 2014 066 709

## Description

### TECHNICAL FIELD

The present disclosure relates to a surgical probe as set forth in claim 1.

### BACKGROUND OF THE DISCLOSURE

For a variety of surgical procedures, for instance in hand surgery, and for more particular instances carpal tunnel, trigger finger or de Quervain's tenosynovitis release, or comparable surgical procedures, probes are required which may be introduced into a narrow longitudinal lumen of the body. Surgery is performed essentially inside said lumen. To this extent, for instance from US 8,523,891, WO 2008/098251, US 5,306,284 and US 8,951,273 surgical probes are known which comprise a longitudinal housing, the probe extending from a proximal end to a distal end. The proximal end is generally provided to be operatively connected to an instrument hand piece. A shaft of the housing is generally hollow, with a longitudinal cavity provided therein, extending along a proximal-distal direction. The longitudinal cavity is generally open at a proximal front face and closed at a distal front face. Proximate a distal end of the probe, an aperture is provided at a circumferential position of the housing, or the longitudinal cavity, respectively, through which the longitudinal cavity is open to the outside of the probe. A blade with a cutting edge is displaceably disposed in the cavity proximate a distal end of the cavity and proximate the aperture. The blade is generally displaceable between a retracted position in which the blade is entirely comprised within the housing, and an operative position in which the cutting edge of the blade is at least partially provided outside the housing. Thus, with the blade in the retracted position a shaft of the probe may be moved inside a body lumen to a surgical site where an incision is to be performed without the blade incising. Once at the intended position, the blade may be actuated in displacing it to the operative position, such that the surgical procedure may be performed. The blade may then be retracted again to remove the probe without causing any harm at locations where no incision is intended to be performed.

Appropriate means to provide a view of the surgical site, that is, a view to a position where the cutting edge is located when the blade is in the operative position, may advantageously be provided.

The cross sectional size of the shaft of the probe is limited in order to provide access to the body lumen, and is typically in a range of some millimeters.

It is apparent that an actuation transmission mechanism is required in order to displace the distally arranged blade as required, wherein the actuation transmission mechanism transmits an actuation action from the proximal end of the probe to the blade, such as to extend the blade and to position at least a part of the cutting edge outside the probe housing, and to retract it in another aspect. An actuation transmission mechanism is for instance described in US 8,951,273.

The actuation transmission mechanism disclosed therein comprises a considerable number of functionally interconnected members, which are linked to each other by tiny pivoting connection elements. As is readily appreciated, these mechanisms are expensive to assemble and provide inside the cavity of the housing with a restricted cross sectional dimension. Moreover, there might be an imminent danger of said miniature pivoting connections blocking or otherwise failing, which might force replacement of the probe, and in turn extension of the surgery duration and additional trauma to the patient. It is moreover apparent that a complex assembly as disclosed in US 8,951,273 may be tough to clean after assembly.

In other aspects, such as described in US 8,523,891, the cross-sectional shape of the shaft of the probe may be of some significance.

Further surgical probes with retractable blades or other cutting or tissue removal devices are for instance known from US2008/0109021, WO2011/017665 and US 2014/0066709.

### LINEOUT OF THE SUBJECT MATTER OF THE PRESENT DISCLOSURE

It is an object of the present disclosure to propose a surgical probe of the kind initially mentioned. In an aspect, an improvement over the art shall be achieved. In a more specific aspect, it is an object of the present disclosure to propose a probe which overcomes the drawbacks of the art, and more in particular of the art described above. In one even more specific aspect, the reliability of the probe shall be improved. In another more specific aspect, the assembly of the probe shall be facilitated.

This is achieved by the subject matter described in claim 1.

Further effects and advantages of the disclosed subject matter, whether explicitly mentioned or not, will become apparent in view of the disclosure provided below.

Accordingly, disclosed is a surgical probe, the surgical probe comprising a housing. The probe extends along a longitudinal extent from a proximal end to a distal end. It is understood that the housing comprises a shaft of the probe. A longitudinal cavity is provided inside the housing and extends along a proximal-distal direction inside the housing. The cavity is open at a proximal front face and is closed at a distal front face thereof. The housing comprises an aperture provided in a distal region of the probe through which the longitudinal cavity is in communication with the exterior of the housing at a circumferential position of the housing, such that the longitudinal cavity is open at a circumferential position through the aperture, and is in communication with the exterior of the housing through the aperture. Circumferential, in this context, is in no way meant to imply a circular cross section. Circumferential is meant to denote any direction which extends across, and in in particular at least essentiallyperpendicular to, the longitudinal, proximal-distal, orientation, or a position which is sideways of the front faces, or proximal-distal extent, of the probe or housing, respectively. The aperture is provided in a distal region of the probe, or of the housing, respectively, that is, in other words, proximate the distal end of the housing, or the probe, respectively, or adjacent said distal end. The aperture does not extend over the distal end, as the longitudinal cavity is closed at the distal front face of the longitudinal cavity, and is accordingly closed at the distal end of the housing and of the probe. A blade and an actuation transmission mechanism are provided inside the housing and in the longitudinal cavity. The blade is disposed distal of the actuation transmission mechanism. In particular, the blade is arranged in a distal section of the longitudinal cavity, that is, proximate or adjacent a distal end of the longitudinal cavity. Further in particular the blade is provided at the same proximal-distal region of the probe, or the housing, or the longitudinal cavity, respectively, than the aperture. Still further in particular, the blade is arranged such that a cutting edge of the blade points towards an outside of the housing. The blade is displaceable in the cavity such as to move the blade between a retracted position in which the blade is entirely comprised inside the housing and an operative position in which the cutting edge of the blade is provided at least partially outside the housing. It is understood that by virtue of this displacement the blade is at least partially moved through the aperture. The actuation transmission mechanism is operatively connected to the blade such that an actuation of the actuation transmission mechanism causes said displacement of the blade. The actuation transmission mechanism may extend along a proximal-distal direction through the longitudinal cavity, in particular with a proximal end of the actuation transmission mechanism being located at or proximate the proximal end of the longitudinal cavity, while a distal end of the actuation transmission mechanism is connected to the blade. The actuation transmission mechanism may in certain aspects be intended to transmit an actuation action applied at the proximal end of the probe to the blade to displace the blade between the retracted and the operative positions. An actuation action, in this context, may be understood as a force and/or a displacement intended to cause displacement of the blade. The blade and the actuation transmission mechanism are integrally provided with each other to form an integral blade unit. That is, the blade and the actuation transmission mechanism are integrally comprised in one single member. The actuation transmission mechanism comprises a thrust transmission section and a flex section. The thrust transmission section is mechanically guided inside the longitudinal cavity with a guide provided on both sides of the thrust transmission section in the plane of the blade, such as to avoid lateral displacement and thus flexing of the thrust transmission section in the plane of the blade. That is to say, in other words, guides are provided which impede a displacement of the thrust transmission section in the plane of the blade, or, in still other words, towards the back and the cutting edge of the blade. In still other words, the thrust transmission section is arranged and provided within the housing with support provided by other structural elements of the probe against displacement in the plane of the blade, or, against bending or bulging.

In further embodiments, the thrust transmission section may, in addition to or instead of providing a guide to the thrust transmission section, be provided with a higher mechanical stiffness against flexion than the flex section. In particular, the thrust transmission section in said embodiments is provided with a higher mechanical stiffness against flexion in the plane of the blade than the flex section.

By virtue of said embodiments the thrust-transmission section is enabled to transmit a pushing force and movement at the proximal end of the blade unit as a mere linear movement, in particular distally oriented along the proximal-distal direction, to the proximal end of the flex section. Accordingly, it will be appreciated that these specific features may ensure that a pushing actuation, for instance a pushing force and/or pushing movement at one longitudinal end of the thrust transmission section, is directly, one by one, transmitted to the opposed end of the thrust transmission section. Embodiments comprising any of these features allow, for instance, a pushing actuation, such as a pushing force and/or pushing movement effected at the proximal end of the probe, to be transmitted in a distally pointing direction and to the flex section, and through the flex section to be transmitted to the blade.

Further, it may in particular be provided that the thrust transmission section is bidirectionally displaceable back and forth along a proximal-distal direction.

It will be appreciated, that, implicitly, also the actuation transmission mechanism is displaceable within the housing, or the longitudinal cavity, respectively, and in particular the actuation transmission mechanism is displaceable along the proximal-distal direction.

In the context of the present disclosure, the terms "orientation" or "direction" shall generally be used to denote the extent of a line. Movements or displacements mentioned along a direction or orientation shall thus be understood to denote a general degree of freedom along this line, allowing bidirectional movements "forward" as well as "backward". Unidirectional movements shall be uniquely denoted by, for instance, "oriented direction", "to" or "towards", or other suitable wording, and will readily be appreciated by the skilled person in the given context.

The skilled person will furthermore readily appreciate that "longitudinal" will be generally understood as aligned with the proximal-distal direction, and will further also be understood as a non-unidirectional expression.

As becomes readily apparent, in the retracted position the cutting edge of the blade is on both lateral sides covered by the housing. Lateral, in this specific context, relates to lateral with respect to directions from the back of the blade to the cutting edge of the blade, and from the distal end of the blade to the proximal end of the blade. It will further be appreciated that said directions define a plane of the blade. The probe may thus be moved inside a lumen of a patient's body without the cutting edge causing trauma. In the operative position, however, at least a part of the cutting edge protrudes from the housing, and thus incisions may by performed.

As noted above, a plane of the blade is defined. It will become readily apparent to the skilled person that the displacement of the blade will at least essentially occur in and be restricted to a displacement in said plane. In particular, the blade may be arranged with its plane at least essentially perpendicular to a cross section of the aperture. It is understood that the blade is arranged and positioned in an aperture of the probe which is formed on a circumferential surface of the probe and extends to and comprises part of the longitudinal cavity. The blade is arranged and positioned such that the cutting edge of the blade points towards the outside of the housing, that is, in the retracted position of the blade the cutting edge points towards the aperture.

In that the blade and the actuation transmission mechanism are integrally provided with each other, joints between several components inside the housing of the probe, along with the risk of potential malfunction and the need for a delicate and expensive assembly, are omitted. Provided that the longitudinal cavity of the housing and the blade unit are suitably adapted to each other with respect to their cross-sectional dimensions, the integral blade unit may be inserted along the proximal-distal direction, and in particular from proximal to distal. It may subsequently, in certain embodiments, be secured inside the housing, or the cavity, respectively, by a retainer member.

In certain embodiments of the surgical probe, the integral blade unit extends distally within the longitudinal cavity from the proximal end, or proximate the proximal end, and in particular extends to the aperture, or to proximate the longitudinal end of the longitudinal cavity. It will be appreciated that in this respect a blade which is positioned at the distal end, or proximate or adjacent the distal end, respectively, may be actuated through the actuation transmission mechanism from the proximal end of the probe.

The skilled person will furthermore readily and fully appreciate the discrimination between the thrust transmission section and the flex section as is understood in the current context and in the sense of the present disclosure. It will be readily understood that those sections are defined by their operational behavior and/or mechanical properties, wherein this may on the one hand be provided by the mechanical properties of the blade unit as such in the respective sections, and/or the arrangement and assembly of the respective sections inside the longitudinal cavity. Generally, it is understood that the thrust transmission section and the flex section are formed and/or received within the housing such that, upon applying a thrust at the proximal end of the blade unit, or, in other words, applying a proximal-distal compressive force on the blade unit inside the probe, or upon applying a bending moment, the flex section will bulge or bend, while the thrust transmission section maintains, at least essentially, its shape. Certain non-limiting instances how this may be achieved are outlined in more detail below. Generally, to name some conceivable embodiments, the thrust transmission section may exhibit a larger inherent bending or bulging stiffness than the flex section, and/or may be supported in the housing against bulging or bending, while the flex section is arranged and provided to allow bending or bulging.

The thrust transmission section may be provided with a higher inherent bending stiffness in the plane of the blade than the flex section. To that extent, the actuation transmission mechanism may for an instance be provided as a strip of sheet material, in particular as a strip of sheet metal. In first exemplary embodiments, the sheet material in the thrust transmission section may be provided with at least one of a larger thickness and/or a three-dimensional shaping with longitudinally extending three-dimensional structures. It is appreciated that these structures may be referred to as stiffening structures, and may for instance be provided as longitudinally extending ribs.

In further exemplary embodiments wherein the actuation transmission mechanism is provided as a strip of sheet material, and in particular as a strip of sheet metal, the sheet material in the flex section is provided with at least one of a width constriction and/or a longitudinal slot. The features of the aforementioned embodiments may be combined with each other.

It may for a more general instance be stated that the comparatively higher mechanical stiffness in the thrust transmission section may be provided in providing a larger cross section in the thrust transmission section than in the flex section, or, a relative reduction of the cross section in the flex section, or in providing geometric stiffening structures in the thrust transmission section, or a combination thereof.

In particular, in embodiments where the actuation transmission mechanism is provided as a strip of sheet material, it is appreciated that the plane of the strip of sheet material, that is, the plane in which the strip of sheet material extends, is provided at least essentially perpendicular to the plane of the blade. In this respect, it will further be readily appreciated that the flex section of the actuation transmission mechanism allows displacement of the blade at least predominantly in the plane of the blade.

The blade unit may in this respect be manufactured from a single piece of sheet material. A strip of sheet material may be processed such as to exhibit the general shape of the actuation transmission mechanism and the blade, and in a transition section between the actuation transmission mechanism and the blade the sheet material may be twisted such as to twist the actuation transmission mechanism and the blade out of the common plane of the sheet material and provide them with the respective planes at least essentially perpendicular to each other. As will be appreciated, a further grinding or other suitable processing step may be performed in order to provide a sharp cutting edge of the blade, which may be performed before or after the twisting step.

At a distal end of the blade unit the blade may be laterally guided against displacement perpendicular to the plane of the blade. To this extent, a guidance slot may be provided inside the housing and in particular at a distal end of the aperture, wherein the blade is, in particular partially and more in particular with at least a part of its back, contained and guided in said guidance slot. As will be appreciated, this guidance feature allows the blade to displace exclusively in the plane of the blade. Upon a displacement which is transmitted to the blade via the actuation transmission mechanism, the lateral guidance will prevent the blade from tilting laterally.

A guide track may be provided, wherein the guide track is shaped such that upon a longitudinal displacement, that is a displacement along the proximal-distal direction, of the blade unit in a distally oriented direction the cutting edge of the blade is at least partially displaced through the aperture and outside the housing. In particular, the back of the blade may be supported against the guide track. For instance, a ramp may be provided inside the housing, at a delimiting wall of the longitudinal cavity, and at a distal end of the aperture. In particular, the ramp may be sloped from inside the housing to the open aperture. That is, a depth measured from the aperture to a bottom of the ramp decreases from proximal to distal. The ramp may be linearly sloped, or may exhibit a curved slope. A curved slope may in particular be progressively sloped from proximal to distal. Due to the guidance by a guide track, a distally oriented displacement of the blade will result in a displacement of the blade from inside the housing towards the aperture and eventually at least partly out of the aperture. In other words, the named guide track causes a longitudinal displacement of the blade to result in a transition of the blade between the retracted position and the operative position.

Said guide track may in particular be provided at the bottom of a slot which in turn is provided to guide the blade against displacement perpendicular to the plane of the blade.

The back of the blade may be provided with an at least essentially tangential transition to the flex section of the actuation transmission mechanism, and the back of the blade may be progressively curved towards the aperture and/or the outside of the housing, respectively, following a direction from said transition to a distal end of the blade.

In certain embodiments of the herein described surgical probe, at least one of a camera and/or an image transferring device is provided inside the housing, wherein a field of view is directed to a position of the edge of the blade when the blade is in the operative position. This allows visual observation of a potential surgical site. The surgeon may accordingly control the position of the probe inside a lumen of a body, and may have an indication of where the body tissue will be incised upon actuation of the blade, and perform an optical in-situ inspection of while incising.

In further aspects, a washer may be provided at the proximal end of the blade unit. The washer is arranged across the proximal-distal direction of the probe. In particular, the washer may be provided integral with the blade unit. The washer may serve to receive an actuation force. In instances, the washer may be provided as an annular member, providing an aperture, in particular a central aperture. The optical arrangement for providing the visual inspection capability of the surgical site, as lined out above, may for instance extend through said central aperture.

At the proximal end of the probe, a connection interface may be provided which enables the probe to be connected to an instrument handle. A releasable operative connection between a proximal end of the blade unit and an actuation means of the instrument handle may be provided such that the probe may be operated and in particular the blade may be moved between the retracted and the operative positions by a manipulation of respective elements of the instrument handle.

In another aspect, a surgical instrument system is disclosed, comprising a surgical probe as herein disclosed and an instrument handle, wherein the surgical probe and the instrument handle are releasably connectable to each other. In still a further aspect, a surgical instrument is disclosed, comprising a surgical probe as herein disclosed and an instrument handle, wherein the surgical probe and the instrument handle are releasably connected to each other. It is understood that the instrument handle may comprise an actuator means, intended to be manipulated by an operator, and operatively connectable, or connected, respectively, to the actuation transmission mechanism of the integral blade unit. Likewise, means may be provided in the instrument handle for transferring an image, or a signal representative of an image, respectively, from an image transferring device or a camera of the probe to an image display device.

As noted, in certain aspects, and independently from the surgical probe initially mentioned and set forth herein, the cross section of a shaft of the probe, wherein the shaft is intended for being inserted into a body lumen, may be of a certain significance. For instance, US 8,523,891 discloses a certain cross sectional shape of the shaft of the probe which is allegedly beneficial for displacing soft tissue with reduced interference with the probe on the one hand, and reduced trauma to the soft tissue due to the displacement and introduction of the probe on the other hand. The embodiments disclosed in the cited document comprise a flat top, with the cross sectional lateral dimension tapering or being constant from the top to the bottom, and in any case at least not increasing in width below the top.

The embodiments disclosed below with respect to the cross section of the shaft of the probe may be provided in combination with one or more of the features described above related to the actuation of the blade, or without them, that is, in combination with or independent of the subject matter disclosed above.

An embodiment is disclosed herein wherein the cross section of the shaft of the probe is generally prolate or oblong, with a longer dimension between a top and a bottom of the shaft, and the shorter dimension between the lateral sides of the shaft. Top and bottom in this respect are not to be understood in a geodetic sense. Following a nomenclature widely used in the technical field, the top of the probe or shaft is defined as the circumferential side on which a window or aperture is provided through which the surgical blade may be extended into an operative position. In the embodiment disclosed herein, the cross section of the shaft exhibits at least a section in which the side walls of the shaft diverge from the top towards the bottom. In other words, there is a part of the cross section present in which the width of the cross section, measured between lateral side walls of the shaft, increases towards the bottom, or, tapers towards the top. In an instance, the shaft of the probe is generally oval in cross section, wherein the straight sections of the perimeter of the cross section are provided on the lateral walls. In another instance, the shaft of the probe is generally egg-shaped in cross section. Straight sections of the perimeter of the egg-shaped cross section may be provided on lateral walls, and may in particular taper towards the bottom. In further instances, the shaft may be provided with a cross section in which the generally oval or the generally egg-shaped cross section is truncated at at least one of the bottom or top, and the perimeter of the cross section comprises a linear section at at least one of the top and the bottom. In this respect, the shaft may comprise a flat top and/or a flat bottom surface. The cross section may then be said to resemble the shape of a rectangle or a trapezoid, wherein the transition between the sides is provided by arcuate geometries, such that the rectangle or trapezoid may be said to be provided with smooth, rounded "corners". In more specific aspects, the width of the flat top of the shaft is 80% or less, more in particular 70% or less, and even more in particular 60% or less, 50% or less, or 40% or less of the maximum width of the cross section. Again, reference is made to the meaning of "top" and "bottom" in the present context, and it will be readily appreciated that a lateral extent is defined, or a width is measured, perpendicular to the top-bottom extent, or height of the shaft.

A ratio of maximum height and maximum width, that is, maximum height divided by maximum width of the shaft cross section, may be larger than or equal to 1.05 and more in particular larger than or equal to 1.10. In another aspect, said ratio of maximum height and maximum width, that is, maximum height divided by maximum width of the shaft cross section, may be smaller than or equal to 1.30 and more in particular smaller than or equal to 1.25 and even more in particular smaller than or equal to 1.20 or even smaller than or equal to 1.15.

It is understood that the cross sectional shape of the shaft, which tapers towards the top in a top section, is well suited to embrace internal functional components of the probe, such as for instance a camera or other optics for allowing a visual inspection of the surgical site. At the same time, the cross sectional dimension of the probe, or, in another aspect, the housing is minimized in that the cross sectional shape is generally adapted to that of the internal functional components. Likewise, the oblong shape allows the actuation mechanism and further internal functional components to be provided alongside each other inside a housing of a minimum possible cross section.

It is understood that the features and embodiments disclosed above may be combined with each other. It will further be appreciated that further embodiments are conceivable within the scope of the present disclosure and the claimed subject matter which are obvious and apparent to the skilled person.

### BRIEF DESCRIPTION OF THE DRAWINGS

The subject matter of the present disclosure is now to be explained in more detail by means of selected exemplary embodiments shown in the accompanying drawings. The figures show
- Fig. 1: a general view of an exemplary embodiment of a surgical probe as herein disclosed;
- Fig. 2: an exploded view of the probe of figure 1;
- Fig. 3: an exemplary embodiment of an integral blade unit;
- Fig. 4: the tip or distal end of the probe with the blade in a retracted position;
- Fig. 5: the tip or distal end of the probe with the blade in a retracted position in a sectional view;
- Fig. 6: the tip or distal end of the probe with the blade in an extended or operative position;
- Fig. 7: the tip or distal end of the probe with the blade in an extended or operative position in a sectional view;
- Fig. 8: a side view of the probe depicting some characteristic dimensions;
- Fig. 9: a top view of the probe depicting some characteristic dimensions;
- Fig. 10: a front view of an exemplary embodiment of a probe pointing out the cross section of the shaft of the probe; and
- Fig 11: an exemplary embodiment of a blank for manufacturing an integral blade unit.

It is understood that the drawings are highly schematic, and details not required for instruction purposes may have been omitted for the ease of understanding and depiction. It is further understood that the drawings show only selected, illustrative embodiments, and embodiments not shown may still be well within the scope of the herein disclosed and/or claimed subject matter.

### EXEMPLARY MODES OF CARRYING OUT THE TEACHING OF THE PRESENT DISCLOSURE

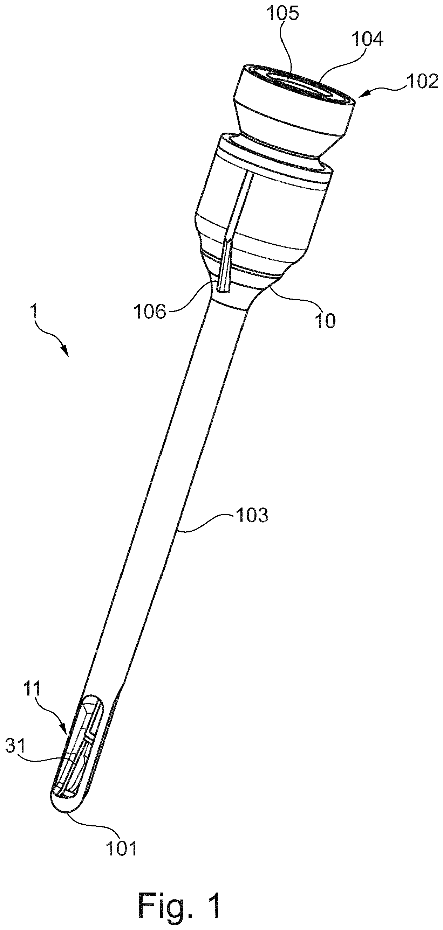

Figure 1 depicts an exemplary embodiment of a surgical probe 1. Surgical probe 1 comprises a distal end or tip 101 and a proximal end 102. A connection interface 104 is provided at the proximal end of surgical probe 1. Through connection interface 104, probe 1 may be releasably connected to an instrument handle. The instrument handle may, in a well-known manner, comprise an actuator means for operating and manipulating an instrument comprising the instrument handle and the probe. The actuation function, and other optional features and functional properties of the handle, will become more apparent in the light of the specification below. Housing 10 comprises and forms a shaft 103 of surgical probe 1 and extends to distal end 101. A longitudinal cavity, not visible in the present depiction, extends inside the housing along a proximal-distal extent. The cavity has an open proximal front face 105 at the proximal end of the surgical probe, and is closed at a distal front face. The longitudinal cavity extends to, or proximate to, the distal end of the probe or of the housing, or of the shaft, respectively. An aperture 11 is provided at a circumferential position of the housing and proximate the distal end of the housing. The longitudinal cavity is open at a circumferential position through aperture 11. In particular, as is the case in the presented embodiment, aperture 11 comprises a longitudinal extent and extends to the distal end of the longitudinal cavity. A blade or knife 31 is provided inside the longitudinal cavity and at the position of the aperture, and is visible and accessible through aperture 11. The blade is shown in a retracted position in which it is entirely comprised within housing 10, and may be at least partly be moved out of the housing and into an operative position, as will become apparent in view of the description below. A cutting edge of blade 31 points towards the aperture, or the outside of the housing, when the blade is in the retracted position as shown in figure 1. In the operative position the cutting edge will at least partly be located outside the housing. The retracted and operative positions of blade 31 are outlined in more detail below. Shaft 103 of the housing is intended to be introduced into a patient's body, whereby the distal part of the probe, including the aperture and the blade, would become invisible. A pointer element 106 is thus provided to indicate the circumferential position of the aperture, and of the cutting edge of blade 31.

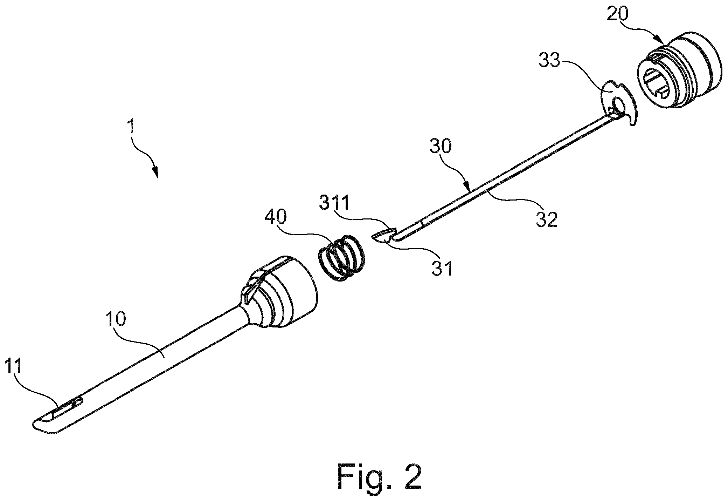

Figure 2 depicts an exploded view of surgical probe 1. Essentially, probe 1 comprises housing 10, integral blade unit 30, proximal end piece 20, and spring 40. In an assembled state, blade unit 30 is slidingly, with a displacement enabled along a proximal-distal direction, received inside housing 10, with blade 31 of integral blade unit 30 provided at aperture 11, as was shown in connection with figure 1. Integral blade unit 30 further comprises an actuation transmission mechanism 32. Blade 31 is connected with its back and at a proximal end of the blade to a distal end of actuation transmission mechanism 32, while cutting edge 311 cantilevers from actuation transmission mechanism 32. At the proximal end of integral blade unit 30, washer 33 is provided across the proximal-distal direction. Washer 33 is an optional feature, and is provided to receive an actuation action for instance from an instrument handle to which the surgical probe may be releasably connected. Blade 31, actuation transmission mechanism 32 and washer 33 are provided integrally with each other to form an integral blade unit. It is understood that generally integral blade unit 30 may be manufactured as an integral workpiece, as is outlined in more detail below. In other instances, integral blade unit 30 may be assembled, for instance, while not limiting, in welding or soldering the blade to the transmission actuation mechanism. It is however decisive that the blade unit is provided as one single integral one-piece member, with no joints or the like required to transmit an actuation action form the proximal end of the surgical probe to the blade. That means, blade unit 30 is received in housing 10 as one single integral piece without the need for assembly inside housing 10. Spring 40 is provided to abut a distal side of washer 33 at the proximal end of spring 40, and further to abut at its distal end a counter surface which is for instance provided as an annular, proximally pointing ledge inside housing 10. Upon applying a distally acting pushing force, or actuation force, on the proximal end of blade unit 30, the proximal end of blade unit 30 is distally displaced inside housing 10. Actuation transmission mechanism 32 transmits the actuation action to blade 31 in a manner outlined below, and blade 31 is displaced into the operative position. Due to the displacement of the proximal end of blade unit 30, and consequently washer 33, spring 40 is compressed. Upon releasing the actuation force, spring 40 will displace the proximal end of blade unit 30 in a proximally oriented direction to the previous, non-actuated, released position. Due to the coupling with actuation transmission mechanism 32, blade 31 will return into its released position. End piece 20 serves as a retainer member, and retains blade unit 30 and spring 40 inside housing 10, and moreover provides, at its proximal end, a connection interface for releasably connecting probe 1 to an instrument handle. Washer 33 is provided with a generally annular geometry, exhibiting a central opening. Through said opening, a camera or other suitable device for visualizing a surgical site at which the blade, when in the operative position, incises, may be provided.

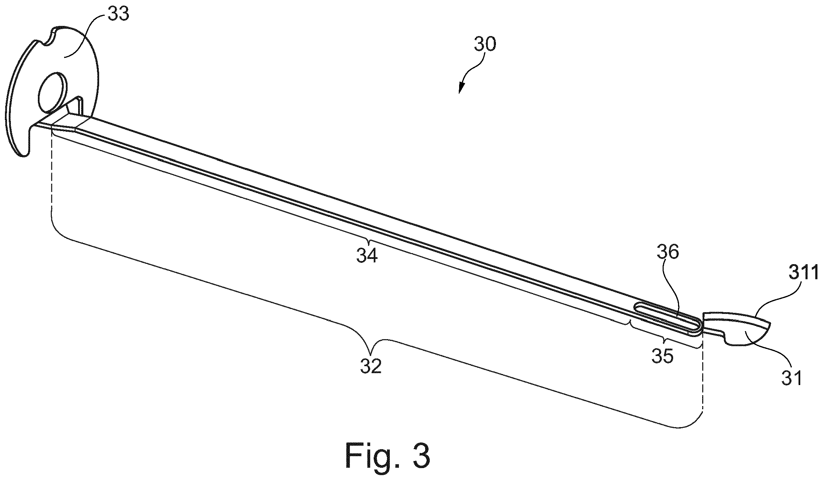

Figure 3 depicts a more detailed view of integral blade unit 30. Actuation transmission mechanism 32 extends from a proximal end of integral blade unit 30 to a proximal end of blade 31. Blade 31 is provided at a distal end of actuation transmission mechanism 32. Blade 31 is connected to the distal end of actuation transmission mechanism 32 at a proximal end of the blade, and at the back of the blade, while cutting edge 311 of blade 31 cantilevers from actuation transmission mechanism. Actuation transmission mechanism 32 comprises thrust transmission section 34 and flex section 35. Flex section 35 is provided distal of thrust transmission section 34, and is interposed between thrust transmission section 34 and blade 31. The discrimination between thrust transmission section and flex section is, in the particular instance shown, provided in that a longitudinally extending slot 36 is provided in a distal section of actuation transmission mechanism 32, that is, adjacent the blade and proximally extending a certain extent. It goes without saying, that in a section of the actuation transmission mechanism where slot 36 is provided the actuation transmission mechanism exhibits a lower inherent stiffness than proximal thereof. Consequently, for instance upon applying a longitudinally acting compressive force on blade unit 30, flex section 35 will bend, while thrust transmission section 34 transmits the compressive force from the proximal end of blade unit 30 to flex section 35. Thus, actuation transmission mechanism 32 is said to comprise thrust transmission section 34 and flex section 35.

It is apparent that blade 31 defines a plane of the blade, extending between the back of the blade and the cutting edge, and the proximal end and a distal end of the blade. It is further apparent, that actuation transmission mechanism 32 is provided as a strip of sheet material, wherein a plane of the sheet material extends at least essentially perpendicular to the plane of the blade. Thus, upon receiving a longitudinally acting compressive force, or a bending momentum, actuation transmission mechanism 32 will, at least predominantly, bend in the plane of the blade. It is apparent that, upon applying a compressive force at the proximal end and the distal end of blade unit 30, actuation transmission mechanism 32 will, at least predominantly, bend or buckle in flex section 35, while thrust transmission section 34 transmits a force from the proximal end of blade unit 30 to the proximal end of flex section 35. In addition to, or in place of, providing longitudinal slot 36, thrust transmission section 34 may be reinforced by longitudinally extending three-dimensional structures, for instance ribs and the like. In other conceivable embodiments it might be the case that a discrimination between the thrust transmission section and the flex section may not be made in view of the blade unit as such, but may only be made when the blade unit is inserted in the housing, and the probe is assembled. To this extent, in the assembled state of the probe, thrust transmission section 34 may be characterized in that it is guided, or supported, against bending or buckling. That is, a support is provided against deformation of a proximal part of actuation transmission mechanism 32, while in a distal part of the actuation transmission mechanism it is provided with at least one degree of freedom to displace in the plane of the blade. Also, an embodiment of the blade unit which allows an immediate distinction between the thrust transmission section and the flex section, said additional support of the thrust transmission section in the assembled probe may be provided, and may further serve to improve the thrust transmission characteristics, as will become apparent in view of the description below.

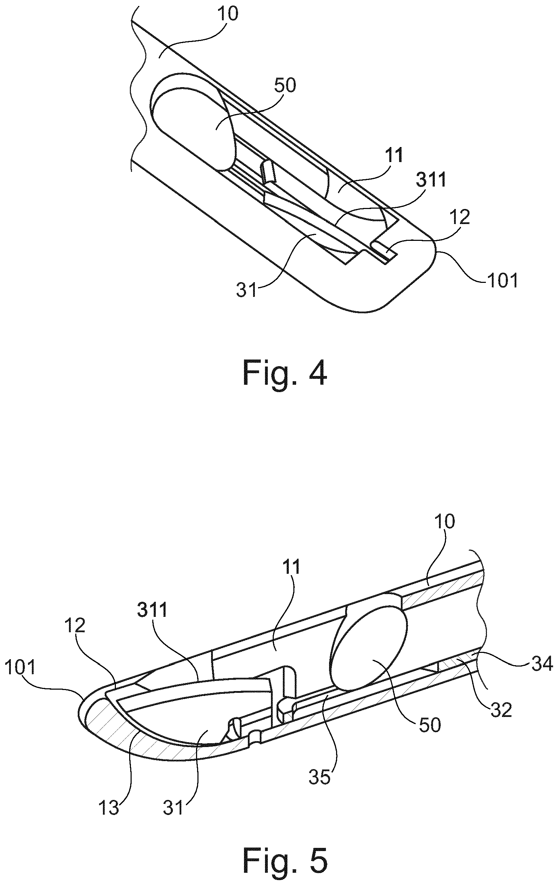

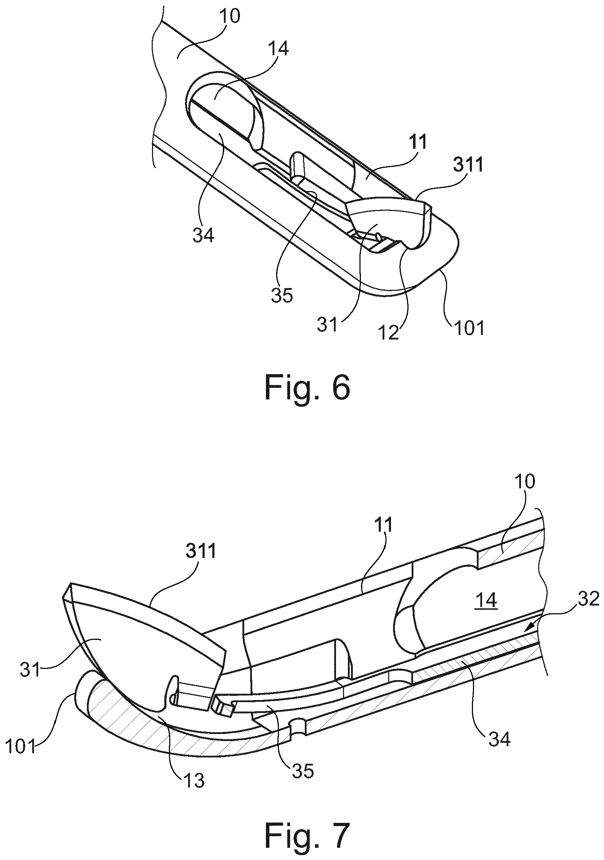

Reference is now made to figures 4 through 7. In figures 4 and 5, the distal portion of a surgical probe according to the present specification is shown with the blade in the retracted position. In figures 6 and 7, the distal portion of the probe is shown with the blade in the extended, operative position. Figures 5 and 7 show longitudinal sections of the depiction of figure 4 and figure 6, respectively. Furthermore, in figures 4 and 5 a camera for visual inspection of a surgical site is shown, while this camera has been omitted in figures 6 and 7, for the sake of a better view of the actuation transmission mechanism.

Turning now to figures 4 and 5, the distal part of a surgical probe is shown. Housing 10 comprises distal tip 101 of the surgical probe. Blade 31 is shown in the retracted position, and is entirely comprised inside housing 10. Such, the probe may be inserted into and be moved inside a patient's body without the blade causing any harm. In the depiction of figure 4, blade 31 is visible through aperture 11. Cutting edge 311 of blade 31 points towards the outside of the probe. As can best be seen in figure 4, the distal end of blade 31 is guided inside a guide slot 12 provided in the housing and at a distal end of aperture 11, or of the longitudinal, proximally-distally extending cavity inside housing 10. Slot 12 is provided such that a lateral displacement of blade 31, that is, a displacement of blade 31 perpendicular to the plane of the blade, is inhibited. In other words, the blade, at least at a distal end, can only be displaced in the plane of the blade. As will be appreciated, blade 31 is supported by the actuation transmission mechanism at the proximal end of the blade, and thus it may be said that a displacement of the entire blade can only take place in the plane of the blade. Further, a camera 50 is provided inside housing 10, and is inserted into the longitudinal cavity of the housing from the proximal end of the probe. The longitudinal cavity is indicated in figures 6 and 7 at reference numeral 14. The camera extends essentially to the proximal end of aperture 11. A view window of the camera is slanted with respect to a proximal-distal axis of the probe, such that the field of view of the camera is directed in a distally oriented direction, and, through aperture 11, to the outside of housing 10, or the probe, respectively. More precisely, the camera is arranged and provided such that the field of view comprises the position of the cutting edge 311 of blade 31 when the blade extends through aperture 11 and is in the operative position, as is shown in figures 6 and 7. Thus, the camera allows a visualization of the position where the blade will incise if moved to the operative position, and allows visual inspection of the surgical site while incising. It is understood, that to this extent camera 50 will also comprise a light source. In the sectional view of figure 5, actuation transmission mechanism 32 is visible, extending inside housing 10 alongside camera 50. Thrust transmission section 34 is guided between housing 10 and camera 50, as is also a part of flex section 35. Upon applying a distally directed force to the proximal end of actuation transmission mechanism 32, the distally acting force will be transmitted through thrust transmission section 34 to flex section 35, and from there to blade 31. At a distal end of the probe, the interior of the probe is provided with a guide track 13 at the bottom of slot 12. Guide track 13 is curved and sloped from inside housing 10 towards aperture 11. Thus, if a distally acting force is transmitted to blade 31, the integral blade unit, comprising actuation transmission mechanism 32 and blade 31, will displace in a distally oriented direction. The back of blade 31 then bears against guide track 13, the blade will follow the shape of guide track 13, and force blade 31 to displace distally and at the same time perpendicular to the proximal-distal extension in the plane of the blade, and, at least partially, out of aperture 11, with cutting edge 311 ahead. As is seen in connection with figures 6 and 7, flex section 35 elastically deforms and bends to allow displacement of blade 31 through aperture 11. As is best seen in connection with figure 7, thrust transmission section 34, due to the higher inherent stiffness, and further due to being guided on both sides in the plane of the blade by the housing and the camera, as is seen in connection with figure 5, remains straight and is thus able to transmit a pushing force to flex section 35. Flex section 35 is provided with a degree of freedom to displace in the plane of the blade. Due to this degree of freedom, and the comparatively lower bending stiffness, flex section 35 is enabled to bend and to allow displacement of blade 31 through aperture 11, as noted above.

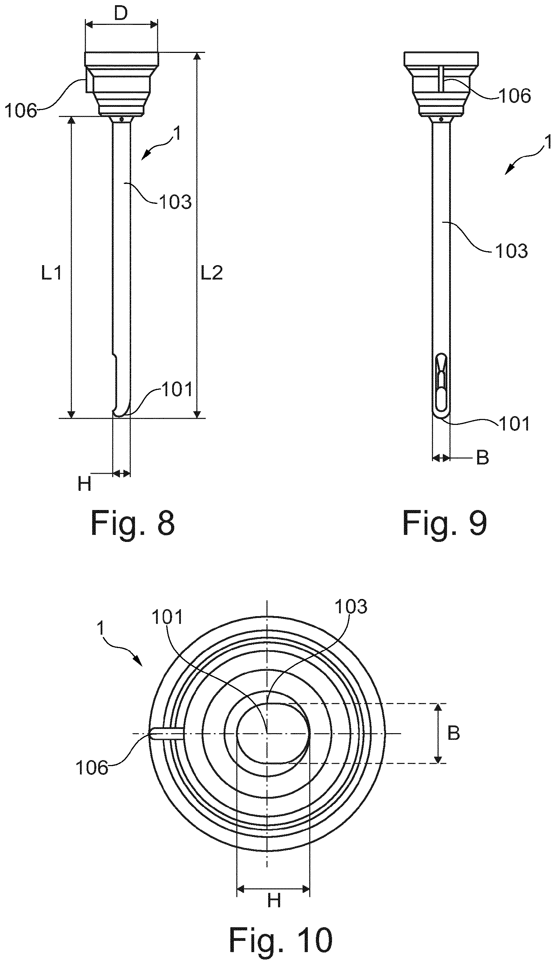

With respect to figures 8 and 9, some exemplary dimensions which the surgical probe 1 may typically exhibit, are provided. A length L1 of shaft 103 may typically be in a range from 60 to 80 millimeters. The total length L2 of the probe may typically range from 80 millimeters to 100 millimeters. A height, or top-bottom extent, H of the shaft may typically range from 3.0 to 5.5 millimeters. A width, or lateral extent, B may typically range from 3.0 to 5.0 millimeters. The proximal cross sectional dimension D of probe 1 may be chosen dependent on the instrument hand piece for use with which the probe is intended.

Figure 10 outlines an exemplary cross-sectional shape of shaft 103. In this instance, shaft 103 exhibits generally the shape of an oval. A ratio H/B of height H to width B, may be larger than or equal to 1.05 and more in particular larger than or equal to 1.10. In another aspect, said ratio may be smaller than or equal to 1.30 and more in particular smaller than or equal to 1.25 and even more in particular smaller than or equal to 1.20 or even smaller than or equal to 1.15. The oblong cross sectional shape of the shaft allows the camera and the actuation transmission mechanism to extend inside the shaft alongside each other, as was pointed out above, while the housing tightly embraces these internal functional components, thus minimizing the cross sectional dimensions of the shaft for a given internal arrangement of components.

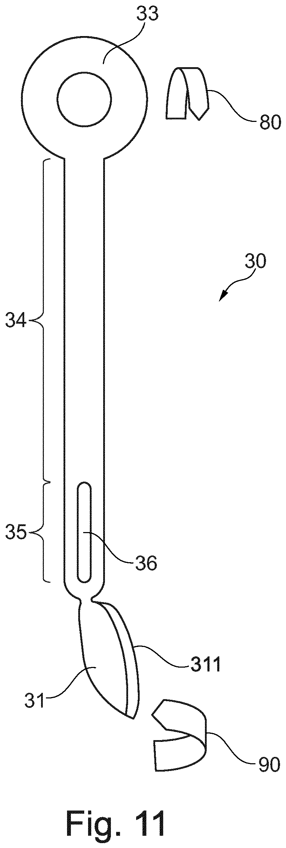

Figure 11 illustrates an exemplary embodiment of manufacturing an integral blade unit 30. A blank workpiece may be punched or otherwise cut, for an instance by laser cutting, out of sheet material, for instance sheet metal. The blank workpiece comprises washer 33, thrust transmission section 34, flex section 35, and blade 31 in one integral piece. It is appreciated, that after punching, or otherwise cutting, the blank workpiece out of a sheet material, all these elements will be provided in one common plane. Longitudinal slot 36 and the aperture of washer 33 may be produced in the initial punching or cutting step, but may also be produced separately at a later stage. Integral blade unit 30 may then be manufactured in bringing washer 33 to an upright position through a bending action as indicated at 80, and twisting blade 31 as indicated at 90, such as to rotate the plane of the blade 90° with respect to the plane of the actuation transmission mechanism, or the thrust transmission section and the flex section, respectively. Cutting edge 311 may be manufactured before or after the cutting step, for instance through grinding.

While the subject matter of the disclosure has been explained by means of exemplary embodiments, it is understood that these are in no way intended to limit the scope of the claimed invention. It will be appreciated that the claims cover embodiments not explicitly shown or disclosed herein, and embodiments deviating from those disclosed in the exemplary modes of carrying out the teaching of the present disclosure will still be covered by the claims.

### LIST OF REFERENCE NUMERALS

- 1: surgical probe
- 10: housing
- 11: aperture
- 12: guide slot
- 13: guide track
- 14: longitudinal cavity
- 20: proximal end piece
- 30: integral blade unit
- 31: blade, knife
- 32: actuation transmission mechanism
- 33: washer
- 34: thrust transmission section
- 35: flex section
- 36: slot
- 40: spring
- 50: camera
- 80: bending
- 90: twisting
- 101: distal end of probe, tip
- 102: proximal end of probe
- 103: shaft
- 104: connection interface
- 105: proximal front face of longitudinal cavity
- 106: pointer element
- 311: cutting edge

- B: width of shaft
- D: proximal cross sectional dimension of probe
- L1: length of shaft
- L2: total length of probe
- H: height of shaft

## Claims

1. A surgical probe (1), the surgical probe comprising a housing(10), the probe extending along a longitudinal extent from a proximal end to a distal end, a longitudinal cavity (14) being provided inside the housing and extending along a proximal-distal direction inside the housing, said cavity being open at a proximal front face and being closed at a distal front face,
the housing comprising an aperture (11) provided in a distal region of the probe and through which the longitudinal cavity is in communication with the exterior of the housing at a circumferential position of the housing, such that the longitudinal cavity (14) is open at a circumferential position through the aperture (11),
a blade (31) comprising a cutting edge (311) and an actuation transmission mechanism (32) being provided inside the housing and in the longitudinal cavity,
the blade (31) being disposed distal of the actuation transmission mechanism (32),
wherein the blade is displaceable in the cavity such as to move the blade between a retracted position in which the blade is entirely comprised inside the housing and an operative position in which the cutting edge of the blade is provided at least partially outside the housing,
the actuation transmission mechanism being operatively connected to the blade such that an actuation of the actuation transmission mechanism causes said displacement of the blade,
wherein the blade (31) and the actuation transmission mechanism (32) are integrally provided with each other to form an integral blade unit (30), and wherein further the actuation transmission mechanism (32) comprises a thrust transmission section (34) and a flex section (35), **characterised by** the thrust transmission section being mechanically guided inside the longitudinal cavity with a guide provided on both sides of the thrust transmission section in the plane of the blade, such as to avoid lateral displacement and thus flexing of the thrust transmission section in the plane of the blade.

2. The surgical probe according to claim 1, wherein the blade unit extends distally within the longitudinal cavity from the proximal end.

3. The surgical probe according to any of the preceding claims, wherein the flex section is provided distal of the thrust transmission section and is interposed between the thrust transmission section and the blade, wherein the thrust transmission section is provided with a higher resistance against flexion than the flex section.

4. The surgical probe according to any of the two preceding claims, wherein the thrust transmission section (34) is provided with a higher inherent bending stiffness in the plane of the blade than the flex section (35).

5. The surgical probe according to the preceding claim, wherein the actuation transmission mechanism (32) is provided as a strip of sheet material, wherein in the thrust transmission section (34) the sheet material is provided with at least one of a larger thickness than in the flex section (35) and/or a three-dimensional shaping with longitudinally extending three-dimensional structures.

6. The surgical probe according to any of the two preceding claims, wherein the actuation transmission mechanism (32) is provided as a strip of sheet material, wherein in the flex section (35) the sheet material is provided with at least one of a width constriction and/or a longitudinal slot (36).

7. The surgical probe according to any of the preceding claims, wherein the blade is, at a distal end of the blade unit, laterally guided against displacement perpendicular to the plane of the blade.

8. The surgical probe according to the preceding claim, wherein the blade is guided in a guidance slot (12) provided inside the housing and in particular at a distal end of the aperture (11).

9. The surgical probe according to any of the preceding claims, wherein a guide track (13) is provided, wherein the guide track is shaped such that upon a longitudinal displacement of the blade unit in a distally oriented direction the cutting edge (311) of the blade is at least partially displaced through the aperture (11) and out of the housing.

10. The surgical probe according to any of the preceding claims, wherein the back of the blade is provided with an at least essentially tangential transition to the flex section of the actuation transmission mechanism, and the back of the blade in the distally oriented direction being progressively curved towards the aperture and/or the outside of the housing, respectively.

11. The surgical probe according to any of the preceding claims, wherein at least one of a camera (50) and/or an image transferring device is provided inside the housing (10), wherein a field of view is directed to a position of the cutting edge (311) of the blade when the blade is in the operative position.

12. The surgical probe according to any of the preceding claims, wherein a connection interface is provided at a proximal end of the probe.

13. The surgical probe according to any of the preceding claims, wherein an outer cross section of a shaft (103) of the probe is generally oblong, a top of the shaft being defined by the circumferential position of the aperture (11), wherein the longer dimension of the oblong cross section is provided between the top and the bottom of the shaft, and wherein the cross section exhibits at least a section in which the side walls of the shaft diverge from the top towards the bottom

14. A surgical instrument system, comprising a surgical probe according to any of the preceding claims and an instrument handle, wherein the surgical probe and the instrument handle are releasably connectable to each other.

## Patentansprüche

1. Chirurgische Sonde (1), wobei die chirurgische Sonde ein Gehäuse (10) umfasst, wobei sich die Sonde entlang einer Längsausdehnung von einem proximalen Ende zu einem distalen Ende erstreckt, wobei ein längslaufender Hohlraum (14) innerhalb des Gehäuses vorgesehen ist und sich entlang einer Proximal-Distal-Richtung innerhalb des Gehäuses erstreckt, wobei der Hohlraum an einer proximalen Stirnseite offen ist und an einer distalen Stirnseite geschlossen ist,
wobei das Gehäuse eine Öffnung (11), die in einem distalen Bereich der Sonde vorgesehen ist und durch die der längslaufende Hohlraum in Verbindung mit der Außenseite des Gehäuses steht, an einer Umfangsposition des Gehäuses umfasst, sodass der längslaufende Hohlraum (14) an einer Umfangsposition durch die Öffnung (11) offen ist, wobei eine Klinge (31), die eine Schneidkante (311) umfasst, und ein Betätigungsübertragungsmechanismus (32) innerhalb des Gehäuses und in dem längslaufenden Hohlraum vorgesehen sind,
wobei die Klinge (31) distal des Betätigungsübertragungsmechanismus (32) angeordnet ist,
wobei die Klinge in dem Hohlraum verschiebbar ist, um die Klinge zwischen einer zurückgezogenen Position, in der sich die Klinge vollständig innerhalb des Gehäuses befindet, und einer Betriebsposition, in der die Schneidkante der Klinge zumindest teilweise außerhalb des Gehäuses vorgesehen ist, zu bewegen,
wobei der Betätigungsübertragungsmechanismus mit der Klinge wirkverbunden ist, sodass eine Betätigung des Betätigungsübertragungsmechanismus die Verschiebung der Klinge bewirkt,
wobei die Klinge (31) und der Betätigungsübertragungsmechanismus (32) einstückig miteinander vorgesehen sind, um eine einstückige Klingeneinheit (30) auszubilden, und wobei der Betätigungsübertragungsmechanismus (32) ferner einen Schubkraftübertragungsabschnitt (34) und einen Biegeabschnitt (35) umfasst, **dadurch gekennzeichnet, dass** der Schubkraftübertragungsabschnitt innerhalb des längslaufenden Hohlraums mechanisch mit einer Führung geführt wird, die auf beiden Seiten des Schubkraftübertragungsabschnitts in der Ebene der Klinge vorgesehen ist, um eine seitliche Verschiebung und somit ein Biegen des Schubkraftübertragungsabschnitts in der Ebene der Klinge zu vermeiden.

2. Chirurgische Sonde nach Anspruch 1, wobei sich die Klingeneinheit innerhalb des längslaufenden Hohlraums distal von dem proximalen Ende erstreckt.

3. Chirurgische Sonde nach einem der vorangehenden Ansprüche, wobei der Biegeabschnitt distal des Schubkraftübertragungsabschnitts vorgesehen ist und zwischen dem Schubkraftübertragungsabschnitt und der Klinge angeordnet ist, wobei der Schubkraftübertragungsabschnitt mit einer höheren Widerstandsfähigkeit gegenüber Biegung versehen ist als der Biegeabschnitt.

4. Chirurgische Sonde nach einem der zwei vorangehenden Ansprüche, wobei der Schubkraftübertragungsabschnitt (34) mit einer höheren inhärenten Biegesteifigkeit in der Ebene der Klinge versehen ist als der Biegeabschnitt (35).

5. Chirurgische Sonde nach dem vorangehenden Anspruch, wobei der Betätigungsübertragungsmechanismus (32) als ein Streifen aus Plattenmaterial vorgesehen ist, wobei in dem Schubkraftübertragungsabschnitt (34) das Plattenmaterial mit mindestens einem aus einer größeren Dicke als in dem Biegeabschnitt (35) und/oder einer dreidimensionalen Formung mit sich längs erstreckenden dreidimensionalen Strukturen versehen ist.

6. Chirurgische Sonde nach einem der zwei vorangehenden Ansprüche, wobei der Betätigungsübertragungsmechanismus (32) als ein Streifen aus Plattenmaterial vorgesehen ist, wobei in dem Biegeabschnitt (35) das Plattenmaterial mit mindestens einem aus einer Breitenverengung und/oder einem längslaufenden Schlitz (36) versehen ist.

7. Chirurgische Sonde nach einem der vorangehenden Ansprüche, wobei die Klinge an einem distalen Ende der Klingeneinheit seitlich gegen eine Verschiebung senkrecht zur Ebene der Klinge geführt wird.

8. Chirurgische Sonde nach dem vorangehenden Anspruch, wobei die Klinge in einem Führungsschlitz (12) geführt wird, der innerhalb des Gehäuses und insbesondere an einem distalen Ende der Öffnung (11) vorgesehen ist.

9. Chirurgische Sonde nach einem der vorangehenden Ansprüche, wobei eine Führungsbahn (13) vorgesehen ist, wobei die Führungsbahn derart geformt ist, dass bei einer Längsverschiebung der Klingeneinheit in einer distal ausgerichteten Richtung die Schneidkante (311) der Klinge zumindest teilweise durch die Öffnung (11) und aus dem Gehäuse hinaus verschoben wird.

10. Chirurgische Sonde nach einem der vorangehenden Ansprüche, wobei die Rückseite der Klinge mit einem zumindest im Wesentlichen tangentialen Übergang zu dem Biegeabschnitt des Betätigungsübertragungsmechanismus versehen ist und die Rückseite der Klinge in der distal ausgerichteten Richtung hin zu der Öffnung beziehungsweise und/oder der Außenseite des Gehäuses zunehmend gekrümmt ist.

11. Chirurgische Sonde nach einem der vorangehenden Ansprüche, wobei mindestens eines aus einer Kamera (50) und/oder einer Bildübertragungsvorrichtung innerhalb des Gehäuses (10) vorgesehen ist, wobei ein Sichtfeld auf eine Position der Schneidkante (311) der Klinge gerichtet ist, wenn sich die Klinge in der Betriebsposition befindet.

12. Chirurgische Sonde nach einem der vorangehenden Ansprüche, wobei eine Verbindungsschnittstelle an einem proximalen Ende der Sonde vorgesehen ist.

13. Chirurgische Sonde nach einem der vorangehenden Ansprüche, wobei ein äußerer Querschnitt eines Schafts (103) der Sonde im Allgemeinen länglich ist, wobei eine Oberseite des Schafts durch die Umfangsposition der Öffnung (11) definiert ist, wobei die längere Abmessung des länglichen Querschnitts zwischen der Oberseite und der Unterseite des Schafts vorgesehen ist und wobei der Querschnitt zumindest einen Abschnitt aufweist, in dem die Seitenwände des Schafts von der Oberseite hin zu der Unterseite auseinandergehen.

14. Chirurgisches Instrumentensystem, umfassend eine chirurgische Sonde nach einem der vorangehenden Ansprüche und einen Instrumentengriff, wobei die chirurgische Sonde und der Instrumentengriff lösbar miteinander verbindbar sind.

## Revendications

1. Sonde chirurgicale (1), la sonde chirurgicale comprenant un boîtier (10), la sonde se prolongeant le long d'une étendue longitudinale d'une extrémité proximale à une extrémité distale, une cavité longitudinale (14) étant ménagée à l'intérieur du boîtier et se prolongeant le long d'une direction proximale-distale à l'intérieur du boîtier, ladite cavité étant ouverte au niveau d'une face avant proximale et étant fermée au niveau d'une face avant distale,
le boîtier comprenant une ouverture (11) ménagée dans une région distale de la sonde et par le biais de laquelle la cavité longitudinale est en communication avec l'extérieur du boîtier à une position circonférentielle du boîtier, de manière que la cavité longitudinale (14) est ouverte à une position circonférentielle par le biais de l'ouverture (11),
une lame (31) comprenant un bord tranchant (311) et un mécanisme de transmission d'actionnement (32) étant fournis à l'intérieur du boîtier et dans la cavité longitudinale, la lame (31) étant située en position distale du mécanisme de transmission d'actionnement (32),
où la lame est déplaçable dans la cavité de manière à faire passer la lame entre une position rétractée dans laquelle la lame est entièrement comprise à l'intérieur du boîtier et une position opérationnelle dans laquelle le bord tranchant de la lame est situé au moins en partie à l'extérieur du boîtier,
le mécanisme de transmission d'actionnement étant relié fonctionnellement à la lame de manière qu'un actionnement du mécanisme de transmission d'actionnement entraîne ledit déplacement de la lame,
où la lame (31) et le mécanisme de transmission d'actionnement (32) sont intégralement fournis l'un avec l'autre pour former une unité à lame intégrale (30), et où le mécanisme de transmission d'actionnement (32) comprend en outre une section de transmission de la poussée (34) et une section flexible (35), **caractérisée en ce que** la section de transmission de la poussée est guidée mécaniquement à l'intérieur de la cavité longitudinale par un guide fourni des deux côtés de la section de transmission de la poussée dans le plan de la lame de manière à éviter un déplacement latéral et donc le fléchissement de la section de transmission de la poussée dans le plan de la lame.

2. Sonde chirurgicale selon la revendication 1, où l'unité à lame se prolonge distalement à l'intérieur de la cavité longitudinale à partir de l'extrémité proximale.

3. Sonde chirurgicale selon l'une quelconque des revendications précédentes, où la section flexible est située à une position distale de la section de transmission de la poussée et interposée entre la section de transmission de la poussée et la lame, où la section de transmission de la poussée est fournie avec une résistance contre la flexion plus élevée que la section flexible.

4. Sonde chirurgicale selon l'une quelconque des deux revendications précédentes, où la section de transmission de la poussée (34) est fournie avec une rigidité inhérente à la flexion dans le plan de la lame qui est plus élevée que celle de la section flexible (35).

5. Sonde chirurgicale selon la revendication précédente où le mécanisme de transmission d'actionnement (32) est fourni sous la forme d'une bande d'un matériau en feuille où, dans la section de transmission de la poussée (34), le matériau en feuille est fourni avec une au moins parmi une épaisseur plus importante que dans la section flexible (35) et/ou une conformation tridimensionnelle à structures tridimensionnelles qui se prolongent longitudinalement.

6. Sonde chirurgicale selon l'une quelconque des deux revendications précédentes où le mécanisme de transmission d'actionnement (32) est fourni sous la forme d'un matériau en feuille où, dans la section flexible (35), la matériau en feuille est fourni avec une au moins parmi une constriction dans le sens de la largeur et/ou une fente longitudinale (36).

7. Sonde chirurgicale selon l'une quelconque des revendications précédentes, où la lame est guidée latéralement, à une extrémité distale de l'unité à lame, de manière à parer contre un déplacement perpendiculaire au plan de la lame.

8. Sonde chirurgicale selon la revendication précédente, où la lame est guidée dans une fente de guidage (12) située à l'intérieur du boîtier et en particulier à une extrémité distale de l'ouverture (11).

9. Sonde chirurgicale selon l'une quelconque des revendications précédentes, où un rail de guidage (13) est fourni, le rail de guidage ayant une forme telle que lors d'un déplacement longitudinal de l'unité à lame dans une direction orientée distalement, le bord de coupe (311) de la lame est au moins au partie déplacé au travers de l'ouverture (11) et hors du boîtier.

10. Sonde chirurgicale selon l'une quelconque des revendications précédentes, où le dos de la lame est fourni avec une transition au moins essentiellement tangentielle par rapport à la section flexible du mécanisme de transmission d'actionnement, et où le dos de la lame dans la direction orientée distalement est progressivement incurvé vers l'ouverture et/ou l'extérieur du boîtier, respectivement.

11. Sonde chirurgicale selon l'une quelconque des revendications précédentes où un au moins parmi une caméra (50) et/ou un dispositif de transfert d'images est fourni à l'intérieur du boîtier (10), où un champ de visualisation est dirigé sur une position du bord de coupe (311) de la lame quand la lame est dans la position opérationnelle.

12. Sonde chirurgicale selon l'une quelconque des revendications précédentes, où une interface de connexion est fournie à une extrémité proximale de la sonde.

13. Sonde chirurgicale selon l'une quelconque des revendications précédentes, où une section transversale extérieure d'une tige (103) de la sonde est généralement allongée, une partie supérieure de la tige étant définie par la position circonférentielle de l'ouverture (11), où la dimension la plus importante de la section transversale allongée est fournie entre la partie supérieure et la partie inférieure de la tige et où la section transversale présente au moins une section au niveau de laquelle les parois latérales de la tige divergent du haut vers le bas.

14. Système d'instrument chirurgical comprenant une sonde chirurgicale selon l'une quelconque des revendications précédentes et un manche d'instrument, où la sonde chirurgicale et le manche d'instrument peuvent être connectés l'un à l'autre de manière amovible.
